(19) Europäisches Patentamt
European Patent Office
Office européen des brevets

(11) Veröffentlichungsnummer : **0 192 998 B1**

# (12) EUROPÄISCHE PATENTSCHRIFT

(45) Veröffentlichungstag der Patentschrift : **24.01.90**

(51) Int. Cl.$^5$ : **C 07 D277/46**, A 01 N 43/78

(21) Anmeldenummer : **86101342.3**

(22) Anmeldetag : **03.02.86**

## (54) Thiazolylamide, Verfahren zu ihrer Herstellung und ihre Verwendung zur Bekämpfung unerwünschten Pflanzenwuchses.

(30) Priorität : **05.02.85 DE 3503773**

(43) Veröffentlichungstag der Anmeldung : **03.09.86 Patentblatt 86/36**

(45) Bekanntmachung des Hinweises auf die Patenterteilung : **24.01.90 Patentblatt 90/04**

(84) Benannte Vertragsstaaten : **BE CH DE FR GB IT LI NL**

(56) Entgegenhaltungen :
**EP-A- 0 001 727**
**EP-A- 0 006 368**
**EP-A- 0 069 154**

(73) Patentinhaber : **BASF Aktiengesellschaft**
**Carl-Bosch-Strasse 38**
**D-6700 Ludwigshafen (DE)**

(72) Erfinder : **Lange, Arno, Dr.**
**Oberes Geistal 3b**
**D-6702 Bad Dürkheim (DE)**
Erfinder : **Würzer, Bruno, Dr.**
**Rüdigerstrasse 13**
**D-6701 Otterstadt (DE)**
Erfinder : **Meyer, Norbert, Dr.**
**Dossenheimer Weg 22**
**D-6802 Ladenburg (DE)**

**EP 0 192 998 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft Thiazolylamide, Verfahren zu ihrer Herstellung, herbizide Mittel, die diese Verbindungen als Wirkstoff enthalten, sowie Verfahren zur Bekämpfung unerwünschten Pflanzenwuchses mit diesen Verbindungen.

Es ist bekannt, daß Thiazolylamide, die am Heterocyclus gegebenenfalls durch Halogen oder Alkyl substituiert sind, herbizid wirksam sind (DE-A 16 42 352).

Weiterhin sind manche Amide, wie Acet- oder Chloracetamide, die in 4-Stellung am Heterocyclus gegebenenfalls substituierte Alkylreste tragen, als Zwischenprodukte oder pharmazeutisch wirksame Substanzen bekannt (EP-A 1727).

Es wurde gefunden, daß Thiazolylamide der Formel

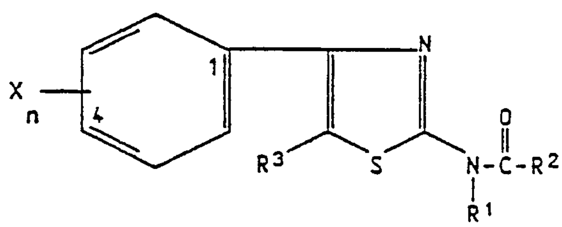

(Ia)

in der

$R^1$ Wasserstoff oder $C_1$-$C_4$-Alkyl,

$R^2$ -Wasserstoff

-$C_3$-$C_7$-Cycloalkyl

-$C_1$-$C_6$-Alkyl, $C_2$-$C_6$-Alkenyl oder $C_2$-$C_6$-Alkinyl, wobei diese Gruppen durch Halogen, $C_1$-$C_4$-Alkoxy, $C_1$-$C_4$-Alkylthio oder Phenoxy substituiert sein können und wobei die Phenoxygruppe ihrerseits durch Halogen oder $C_1$-$C_4$-Alkyl substituiert sein kann,

$R^3$ Wasserstoff, $C_1$-$C_4$-Alkyl oder Halogen,

X Wasserstoff, Halogen, $C_1$-$C_6$-Alkoxy, $C_1$-$C_6$-Halogenalkoxy, $C_1$-$C_6$-Alkyl, $C_1$-$C_6$-Halogenalkyl, $C_3$-$C_6$-Cycloalkyl, $C_1$-$C_6$-Alkylthio, Nitro, Cyano, Benzyloxy, gegebenenfalls durch Halogen substituiertes Phenyl oder gegebenenfalls durch Halogen und/oder Halogenmethyl substituiertes Phenoxy und

n 1, 2, 3 oder 4 bedeuten, herbizid selektiv wirksam sind.

$R^1$ in Formel Ia bedeutet Wasserstoff oder $C_1$-$C_4$-Alkyl, wie Methyl, Ethyl, n-Propyl, i-Butyl, vorzugsweise Wasserstoff; $R^2$ in Formel Ia bedeutet Halogen, $C_3$-$C_7$-Cycloalkyl, einen gegebenenfalls durch Halogen, gegebenenfalls durch Halogen oder $C_1$-$C_4$-Alkyl substituiertes Phenoxy, $C_1$-$C_4$-Alkoxy oder $C_1$-$C_4$-Alkylthio, substituierten $C_1$-$C_6$-Alkyl, $C_2$-$C_6$-Alkenyl- oder $C_2$-$C_6$-Alkinylrest, beispielsweise Methyl, Ethyl, n-Propyl, Isopropyl, t-Butyl, Vinyl, Allyl, Ethinyl, Prop-1-inyl, Propargyl, But-2-inyl, 1-Methyl-prop-2-inyl, But-4-inyl, 1,1-Dimethyl-prop-2-inyl, Cyclopropyl, Cyclobutyl, Cyclopentyl, Cyclohexyl, Methoxymethyl, Ethoxylmethyl, 2-Methoxy-ethyl, 2-Ethoxy-ethyl, Methylthiomethyl, n-Butoxymethyl, Trichlormethyl, 1,2-Dichlorethyl, 2,4-Dichlorphenoxymethyl, 4-Chlorphenoxy-methyl, 2-Methyl-4-chlorphenoxy-methyl, 2,4,5-Trichlorphenoxy-methyl. Bevorzugt sind gegebenenfalls substituierte $C_1$-$C_4$-Alkyl-, $C_2$-$C_4$-Alkenyl- und $C_2$-$C_4$-Alkinylreste für $R^2$. Besonders bevorzugte Reste für $R^2$ sind Ethyl, Cyclopropyl und Isopropenyl.

X in Formel Ia bedeutet Wasserstoff, Halogen, $C_1$-$C_6$-Alkoxy, vorzugsweise $C_1$-$C_4$-Alkoxy, $C_1$-$C_6$-Halogenalkoxy, vorzugsweise $C_1$-$C_4$-Halogenalkoxy, wobei als Halogensubstituenten insbesondere Fluor und/oder Chlor in Betracht kommen, $C_1$-$C_6$-Alkyl, vorzugsweise $C_1$-$C_4$-Alkyl, $C_1$-$C_6$-Halogenalkyl, vorzugsweise $C_1$-$C_4$-Halogenalkyl, wobei als Halogensubstituenten insbesondere Fluor und/oder Chlor in Betracht kommen, $C_3$-$C_6$-Cycloalkyl, $C_1$-$C_6$-Cycloalkyl, $C_1$-$C_6$-Alkylthio, vorzugsweise $C_1$-$C_4$-Alkylthio, Nitro, Cyano, Benzyloxy, gegebenenfalls durch Halogen, wie Chlor, substituiertes Phenyl oder gegebenenfalls durch Halogen, wie Chlor und/oder Halogenmethyl, wie Trifluormethyl, substituiertes Phenoxy, beispielsweise Fluor, Chlor, Brom oder Iod, vorzugsweise Fluor, Chlor oder Brom, Methoxy, Ethoxy, Difluormethoxy, Trifluormethoxy, 3,3,3,2,1,1-Hexafluor-n-propoxy, 2,1,1-Trifluor-2-chlor-ethoxy, Methyl, Ethyl, t-Butyl, Trifluormethyl, Cyclohexyl, Methylthio, Ethylthio, Nitro, Cyano, Benzyloxy, Phenyl, 4-Chlorphenyl, -4Trifluormethylphenyl, 2-Chlor-4-trifluormethylphenoxy, Phenoxy, 4-Chlorphenoxy.

Bevorzugte Thiazolylamide der Formel Ia sind solche, bei denen $R^1$ Wasserstoff und $R^2$ $C_1$-$C_4$-Alkyl, vorzugsweise Ethyl, $C_2$-$C_4$-Alkenyl, vorzugsweise Propenyl, Isopropenyl, oder $C_3$-$C_5$-Cycloalkyl, vorzugsweise Cyclopropyl, bedeuten. Weiterhin bevorzugte Thiazolylamide der Formel Ia sind solche, bei denen X $C_1$-$C_4$-Halogenalkoxy, insbesondere durch Fluor und/oder Chlor substituiertes Alkoxy, bedeutet. Besonders bevorzugt sind solche, bei denen $X_n$ eine $CHF_2O$-oder $CF_3O$-Gruppe in 4-Position ist.

Amide der Formel

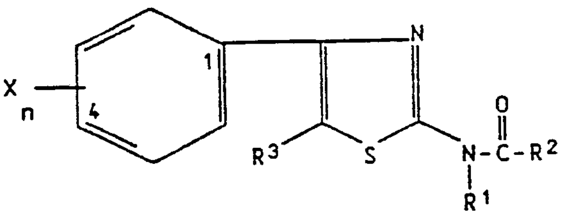

(I)

in der

$R^1$ Wasserstoff oder $C_1$-$C_4$-Alkyl,

$R^2$ $C_2$-$C_4$-Alkyl, $C_2$-$C_4$-Alkenyl, $C_2$-$C_4$-Alkinyl oder $C_3$-$C_7$-Cycloalkyl,

$R^3$ Wasserstoff, $C_1$-$C_4$-Alkyl oder Halogen,

X Halogen, $C_1$-$C_6$-Alkoxy, $C_1$-$C_6$-Halogenalkoxy, $C_1$-$C_6$-Alkyl, $C_1$-$C_6$-Halogenalkyl, $C_3$-$C_6$-Cycloalkyl, $C_1$-$C_6$-Alkylthio, Nitro, Cyano, Benzyloxy, gegebenenfalls durch Halogen substituiertes Phenyl oder gegebenenfalls durch Halogen und/oder Halogenmethyl substituiertes Phenoxy und

n 1, 2, 3 oder 4 bedeuten, sind neu.

Thiazolylamide der Formel I können durch eine Anzahl verschiedener üblicher Verfahrensweisen hergestellt werden. Beispielsweise können gemäß einem im folgenden als Methode A bezeichneten Verfahren Thiazolylamide der Formel I durch Umsetzen von Thiazolylaminen der Formel

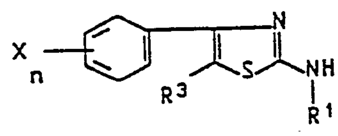

(II)

in der $R^1$, $R^3$ und $X_n$ die obengenannten Bedeutungen haben, mit Säureanhydriden der Formel

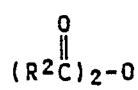

(III)

worin $R^2$ die obenangegebenen Bedeutungen hat, in Gegenwart oder in Abwesenheit eines Katalysators, wie Pyridin, und in Gegenwart oder in Abwesenheit eines Lösungsmittels hergestellt werden.

Weiterhin können die Thiazolylamide der Formel I gemäß einem anderen Verfahren, das im folgenden als Methode B bezeichnet wird, erhalten werden, wobei Thiazolylamine der Formel II mit Säurehalogeniden der Formel

$R^2COHal$ (IV)

wobei $R^2$ die oben angegebenen Bedeutungen hat, und Hal Chlor- oder Brom, vorzugsweise Chlor bedeutet, umgesetzt werden. Die Reaktion wird in einem inerten Lösungsmittel in Gegenwart eines Säureacceptors, wie einer organischen oder anorganischen Base, durchgeführt.

Nach einem weiteren Verfahren C werden Isocyanate der Formel V gemäß der folgenden Gleichung mit Carbonsäuren (VI) umgesetzt:

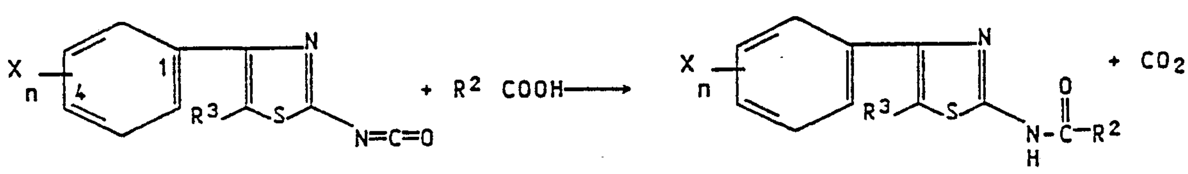

(V) (VI)

wobei $R^2$, $R^3$ und $X_n$ die obengenannte Bedeutung haben. Dieser Syntheseweg führt zu Thiazolylamiden der Formel I, bei denen $R^1$ Wasserstoff ist.

Typische Reaktionsteilnehmer bei der Umsetzung A sind beispielsweise:

a) 4-(4-Difluormethoxyphenyl)-thiazol-2-ylamin mit irgendeinem der folgenden beispielsweise genannten Anhydride: Propionsäureanhydrid, 2-Methyl-pentansäureanhydrid, Cyclopropancarbonsäureanhydrid, Methacrylsäureanhydrid, Isobuttersäureanhydrid.

b) 4-(4-Trifluormethoxyphenyl)-thiazol-2-ylamin mit einem der in a) angegebenen Anhydride.

Die Umsetzung gemäß Methode A kann in Gegenwart oder in Abwesenheit eines inerten organischen Lösungsmittels durchgeführt werden. Das inerte organische Lösungsmittel kann ein Kohlenwasserstoff, wie Ligroin, Benzol, Toluol, ein Ether, wie Dioxan, oder ein chlorierter Kohlenwasserstoff, wie Chlorbenzol, Tetrachlorkohlenstoff, Perchlorethylen, sein. Ebenso können Gemische dieser Lösungsmittel verwendet werden. Anstelle eines Lösungsmittels kann auch in einem Überschuß des Säurehydrids der Formel III umgesetzt werden.

Als Katalysatoren kommen tertiäre Amine in Mengen von 0,01 bis 1,1 Mol, bezogen auf 1 Mol Thiazolylamin der Formel II in Betracht. Es können beispielsweise Picolin, Pyridin, Triethylamin, N-Methylpiperidin und N,N-Dimethylanilin verwendet werden.

Obwohl das Anhydrid der Formel III als Lösungsmittel verwendet und der Überschuß für die weitere Verwendung zurückgewonnen werden kann, ist es im allgemeinen am günstigsten, äquimolare Mengen bei den Reaktionsteilnehmern zu verwenden und ein Lösungsmittel zuzusetzen.

Die Reaktionstemperaturen können über einen weiten Bereich von etwa 0 °C bis zum Siedepunkt des Lösungsmittels (ca. 150 °C) variieren.

Die gemäß Methode B verwendeten Reaktionsteilnehmer sind Phenylthiazolylamine der Formel II und die Säurechloride der Formel IV.

Typische, bei dem Verfahren B verwendete Reaktionsteilnehmer sind :

a) 4-(4-Difluormethoxyphenyl-thiazol-2-ylamine mit einem der folgenden Säurechloride : Acetylchlorid, Propionylchlorid, Isobutyrylchlorid, Methacrylchlorid, 2-Methylpentanoylchlorid, Fluoracetylchlorid, Cyclopropancarbonsäurechlorid.

b) 4-(4-Trifluormethoxyphenyl-thiazol-2-ylamine mit irgendeinem der in a) angegebenen Säurechloride.

Zur Durchführung der Umsetzung B wird das Säurehalogenid in einem Überschuß von 0 bis 50 Mol.% pro Mol II, gegebenenfalls in Gegenwart eines Lösungsmittels und gegebenenfalls in Gegenwart eines Säureacceptors bei einer Temperatur von — 20 bis + 150 °C, vorzugsweise von 0 bis + 80 °C, kontinuierlich oder diskontinuierlich umgesetzt (Houben-Weyl, Methoden der organischen Chemie, Bd. VIII, S. 655 ff (1951)).

Als Säureacceptoren können die üblichen Mittel, beispielsweise Alkalihydroxide, Alkalicarbonate, Alkalialkoholate und tertiäre organische Basen, verwendet werden. Als besonders geeignet seien im einzelnen genannt: Natriumhydroxid, Natriumhydrogencarbonat, Natriumethylat, Natriumcarbonat, entsprechende Kaliumsalze, Triethylamin, Pyridin, Trimethylamin, α-, β-Picolin, Lutidin, N,N-Dimethylanilin, N,N-Dimethylcyclohexylamin, Chinolin, Tri-n-propylamin, Tri-n-butylamin und Acridin, N,N-Dimethyl-, Diethylpiperazin, Methyl-, Ethyl-piperidin, Methyl-, Ethyl-pyrrolidin.

Die Umsetzung C wird durchgeführt, indem man die Reaktionsteilnehmer der Formeln II und V in einem hochsiedenden inerten Lösungsmittel, wie Toluol, Xylol, Chlorbenzol oder Dichlorbenzol unter Rückfluß erhitzt, bis die Entwicklung von Kohlendioxid beendet ist.

Die Isolierung der Endprodukte erfolgt bei allen Verfahrensvarianten entweder bei aus dem Reaktionsgemisch ausgefallenen Stoffen durch Absaugen und anschließende Reinigung durch Waschen, Umkristallisieren bzw. Chromatographieren oder durch Einengen des Reaktionsgemisches unter vermindertem Druck zur Trockne und Reinigung des Rückstandes durch Umkristallisieren bzw. Chromatographieren.

Die als Ausgangsstoffe verwendeten substituierten Thiazolylamine der Formel II sind zum Teil neu. Sie können alle nach an sich bekannten Verfahren hergestellt werden. (J. Chem. Soc. 67, 2242-3 (1945)).

Dabei werden die folgenden Thiazolylamine der Formel (IIa) erhalten :

(Siehe Tabellen Seite 7 ff.)

4-Aryl-2-amino-5-halo-thiazole werden nach der in J. Ind. Chem. Soc. Vol. 36, No. 5 (1959) beschriebenen Methode erhalten.

## Beispiel 1

106 g 4-Hydroxyacetophenon werden in 450 ml Dioxan vorgelegt. Man gibt 156 g NaOH in 390 ml Wasser und 4 g Tetrabutylammoniumiodid zu. Dann wird bei 65 bis 70 °C über 4,5 Stunden Chlordifluormethan bis zur Sättigung eingegast. Nun wird abgesaugt, der Festkörper mit Dichlormethan gewaschen und das Filtrat dreimal mit Dichlormethan extrahiert. Die Dichlormethan-Phasen werden vereinigt, mit Wasser gewaschen, getrocknet, eingeengt und destilliert. Man erhält 127,4 g 4-Difluormethoxyacetophenon ; $KP_{0,1mbar}$ : 78 bis 81 °C.

40 g 4-Difluormethoxyacetophenon und 35,7 g Thioharnstoff werden vorgelegt. Man setzt 61 g Jod zu, wobei die Temperatur auf 40 °C steigt. Dann hält man 6 Stunden bei 100 °C. Bei 100 °C wird Wasser zugetropft, auf Raumtemperatur abkühlen lassen, abgesaugt, mit Ether und verdünnter $NH_3$-Lösung

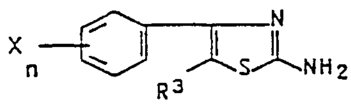

| $X_n$ | $R^3$ | Fp [°C] |
|---|---|---|
| $4-CF_3$ | H | 182-188 |
| H | $CH_3$ | 120-124 |
| $4-OCH_3$ | H | 200-206 |
| $4-NO_2$ | H | >300 |
| $4-CH_3$ | H | 131-134 |
| 4-Cl | H | 163-166 |
| $3,4-Cl_2$ | H | 160-164 |
| $2,4-Cl_2$ | H | 161-163 |
| 2-Cl | H | 137-140 |
| 4-F | H | 109-114 |
| $2,4-F_2$ | H | |
| 3-Br | H | 127-129 |
| 4-Br | H | 175-177 |
| $4-OC_2H_5$ | H | 241-246 |
| $4-OCHF_2$ | H | 113-118 |
| $4-OCF_3$ | H | |
| $4-OC_6H_5$ | H | 116-118 |
| $4-OCH_2-C_6H_5$ | H | 155-159 |
| 3-Cl, 4-(4-Chlorphenoxy) | H | 120-123 |
| $4-SCH_3$ | H | 180-183 |
| 3-Cl, $4-OCH_3$ | H | |
| $4-Cyclo-C_6H_{11}$ | H | 176-180 |
| $4-OCF_2-CHF-CF_3$ | H | 95- 98 |
| $2-OCH_3$, $3,5-Cl_2$ | H | |
| $2,5-Cl_2$ | H | 166-169 |
| $4-OCH_3$ | $CH_3$ | 136-138 |
| $3-OCH_3$ | H | 122-125 |
| $2,4-(OCH_3)_2$ | H | 183-189 |
| $4-(4-CF_3$, $2-Cl-C_6H_3)$ | H | |
| 4-Cl | $CH_3$ | 135-140 |
| $2,4-(OCHF_2)_2$ | H | |
| 2-F | H | |
| $2,3,4-Cl_3$ | H | |
| $2-OCHF_2$, $4-CH_3$ | H | |
| $2-OCHF_2$, 4-Cl | H | |
| 2-Cl, $4-OCHF_2$ | H | |
| $2-CH_3$, $4-OCHF_2$ | H | |

gewaschen, mit Wasser neutral gewaschen und bei 50 °C unter vermindertem Druck getrocknet. Man erhält 33,8 g 4-(4-Difluormethoxyphenyl)-thiazol-2-ylamin.

8,23 g 4-(4-Difluormethoxyphenyl)-thiazol-2-ylamin werden mit 3,7 g Triethylamin in 80 ml Toluol vorgelegt. Es werden 3,46 g Propionsäurechlorid zugetropft, 2 Stunden bei 50 °C und über Nacht bei Raumtemperatur gehalten. Der Festkörper wird abgesaugt, gut mit Wasser gewaschen und über Nacht unter vermindertem Druck bei 50 °C getrocknet. Man erhält 7 g N-Propionyl-[4-(4-difluormethoxyphenyl)-thiazol-2-yl]amin vom Schmelzpunkt 186 bis 188 °C (Verbindung Nr. 45).

Beispiel 2

30 g 4-Methoxyacetophenon werden mit 33,5 g Thioharnstoff versetzt. Man gibt 56 g Iod zu und hält 5 Stunden bei 100 °C. Das Reaktionsgemisch wird auf Wasser ausgetragen, gepulvert und mehrfach mit Ether, dann mit Ammoniaklösung und Wasser gewaschen und getrocknet. Man erhält 37,8 g 4-(4-Methoxyphenyl)-thiazol-2-ylamin vom Schmelzpunkt 194 bis 205 °C.

4,94 g 4-(4-Methoxyhenyl)-thiazol-2-ylamin werden in 60 ml Toluol mit 2,63 g Triethylamin vorgelegt. Man tropft 6,23 g 2,4-Dichlorphenoxyacetylchlorid in 5 ml Toluol zu und läßt über Nacht bei Raumtemperatur reagieren. Es wird abgesaugt, gut mit Wasser gewaschen und getrocknet. Man erhält 6 g N-[4-(4-Methoxyphenyl)-thiazol-2-yl]-(2,4-dichlorphenoxy)-acetamid vom Schmelzpunkt 135 bis 139 °C (Verbindung Nr. 4).

Beispiel 3

18,6 g 4-Difluormethoxyacetophenon werden mit 16,7 g Thioharnstoff und 20 ml Methylchloroform versetzt. Zu der gerührten Maische werden 14,9 g Sulfurylchlorid getropft, wobei die Temperatur auf 37 °C steigt. Man heizt auf 80 bis 90 °C und hält 12 Stunden bei dieser Temperatur.

Bei Raumtemperatur wird abgesaugt, 2 x mit Methylchloroform, 2 x mit Wasser, 3 x mit verd. Ammoniaklösung dreimal mit Wasser gewaschen, trocken gesaugt und unter vermindertem Druck bei 40 °C getrocknet. Man erhält 17,3 g 2-Amino-4-(4-difluormethoxyphenyl)-thiazolin (≙ 71,5 %, Ausbeute vom Schmelzpunkt 116 bis 120 °C.

Bei Raumtemperatur werden 9,7 g 2-Amino-4-(4-difluormethoxyphenyl)-thiazolin in 80 ml Toluol vorgelegt und mit 4,5 g Triethylamin versetzt. Man tropft 4,6 g Cyclopropancarbonsäurechlorid zu, wobei sich die Temperatur von 22 °C auf 42 °C erhöht. Es wird noch eine Stunde bei 45 °C nachgerührt.

Dann läßt man 150 ml Wasser zulaufen, saugt den Festkörper ab und wäscht zweimal mit Wasser. Der Festkörper wird bei vermindertem Druck getrocknet. Man erhält 8,8 g N-Cyclopropylcarbonyl-N-[4-(4-difluormethoxyphenyl)-thiazol-2-yl]-amin vom Fp. 202 bis 203 °C.

Analog können folgende Verbindungen hergestellt werden :

(Siehe Tabellen Seite 7 ff.)

Die Thiazolylamide der Formel Ia können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sicht ganz den den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Toluol, Xylol, Paraffin, Tetrahydronaphthalin, alkylierte Naphthaline oder deren Derivate, z. B. Methanol, Ethanol, Propanol, Butanol, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuel Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctyl-phenolether, ethoxyliertes Isooctylphenol, Octylphenol, Nonyl-

| Verbindung Nr. | $X_n$ | $R^3$ | $R^2$ | $R^1$ | Fp [°C] |
|---|---|---|---|---|---|
| 1 | 4-$CF_3$ | Cl | $C_2H_5$ | H | |
| 2 | 4-(4-Trifluormethyl-2-chlor-phenoxy) | Cl | $C_2H_5$ | H | |
| 3 | H | $CH_3$ | $C_2H_5$ | H | 176-178 |
| 4 | 4-$OCH_3$ | H | 2,4-$Cl_2$-$C_6H_3$-O-$CH_2$- | H | 135-139 |
| 5 | 4-(4-Trifluormethyl-2-chlor-phenoxy) | H | $C_2H_5$ | H | |
| 6 | 4-$NO_2$ | H | $CH_3$ | H | |
| 7 | 4-$CF_3$ | H | $C_2H_5$ | H | 219-224 |
| 8 | 4-$CH_3$ | H | $C_2H_5$ | H | 179-183 |
| 9 | 4-$OCH_3$ | H | $C_2H_5$ | H | 175-180 |
| 10 | 4-$OCH_3$ | H | Cyclo-$C_3H_5$ | H | 165-180 |
| 11 | H | H | 2,4-$Cl_2$-$C_6H_3$-O-$CH_2$- | H | 140-142 |
| 12 | 4-$CH_3$ | H | n-$C_3H_7$ | H | |
| 13 | 4-Cl | H | $C_2H5$ | H | 209-211 |
| 14 | 4-Cl | H | Cyclo-$C_3H_5$ | H | 218-227 |
| 15 | 3-Cl | H | n-$C_4H_9$ | $CH_3$ | |
| 16 | 3,4-$Cl_2$ | H | $C_2H_5$ | H | 198-200 |
| 17 | 3,4-$Cl_2$ | H | $CH_3O$-$CH_2$ | H | |
| 18 | 4-F | H | $C_2H_5$ | H | 182-184 |
| 19 | 4-F | H | 2,4-$Cl_2$-$C_6H_3$-O-$CH_2$- | H | 168-173 |
| 20 | 4-F | H | Cyclo-$C_3H_5$ | H | 201-208 |
| 21 | 3-Br | H | Cl-$CH_2$ | $C_2H_5$ | |
| 22 | 3-Br | H | $CH_3$ | $C_3H_7$ | |
| 23 | 3-Br | H | $CH_2$=CH- | H | |
| 24 | H | $CH_3$ | Cyclo-$C_3H_5$ | H | |

| Verbindung Nr. | $X_n$ | $R^3$ | $R^2$ | $R^1$ | Fp [°C] |
|---|---|---|---|---|---|
| 25 | H | H | $CH_2=C(CH_3)-$ | H | |
| 26 | H | Br | $C_2H_5$ | H | |
| 27 | $3-OCH_3$ | H | $C_2H_5$ | H | |
| 28 | $3-OCH_3$ | Cl | $C_2H_5$ | H | |
| 29 | $3-OCH_3$ | Br | $C_2H_5$ | H | |
| 30 | $4-OC_2H_5$ | H | $C_2H_5$ | H | |
| 31 | $4-OC_2H_5$ | H | $C_2H_5$ | $CH_3$ | |
| 32 | $4-OC_2H_5$ | H | $Cl-CH_2-$ | H | |
| 33 | $4-OCH_3$ | $CH_3$ | Cyclo-$C_6H_{11}$ | H | |
| 34 | $4-OCH_3$ | $CH_3$ | $CH\equiv C-$ | H | |
| 35 | $4-OCH_3$ | $CH_3$ | $CH_3-S-CH_2$ | H | |
| 36 | $4-OC_6H_5$ | H | $C_2H_5$ | H | |
| 37 | $4-OC_6H_5$ | H | $CH_2=C(CH_3)-$ | H | |
| 38 | $4-OC_6H_5$ | H | Cyclo-$C_5H_9$ | H | |
| 39 | $4-OCH_2C_6H_5$ | H | $C_2H_5$ | H | |
| 40 | $4-OCH_2C_6H_5$ | Cl | $C_2H_5$ | H | |
| 41 | $4-OCH_2C_6H_5$ | H | $2-Cl,4-CH_3,C_6H_3-OCH_2$ | H | |
| 42 | 2-Cl | H | $2,4-Cl_2-C_6H_3-O-CH_2-$ | H | 160-162 |
| 43 | 2-Cl | H | $C_2H_5$ | H | 102-107 |
| 44 | 2-Cl | H | Cyclo-$C_3H_5$ | H | 128-130 |
| 45 | $4-OCHF_2$ | H | $C_2H_5$ | H | 186-188 |
| 46 | $4-OCHF_2$ | H | $CH_2Cl$ | H | 126-128 |
| 47 | $4-OCHF_2$ | H | Cyclo-$C_3H_5$ | H | 202-203 |
| 48 | 3-Cl, 4-(4-Chlorphenoxy) | H | $C_2H_5$ | H | 208-210 |

| Verbindung Nr. | $X_n$ | $R^3$ | $R^2$ | $R^1$ | Fp [°C] |
|---|---|---|---|---|---|
| 49 | 4-(4-Chlorphenoxy) | H | $C_2H_5$ | H | |
| 50 | 3, 4-(4-Chlorphenoxy) | H | $Cl-CH_2-$ | H | |
| 51 | $4-CH_3$ | H | $Br-CH_2$ | H | |
| 52 | $4-SCH_3$ | H | $2,4-Cl_2-C_6H_3-O-CH_2-$ | H | 164-165 |
| 53 | $4-SCH_3$ | H | $C_2H_5$ | H | 205-207 |
| 54 | $4-SCH_3$ | H | $CH_2=C(CH_3)-$ | H | |
| 55 | 3-Cl, $4-OCH_3$ | H | $C_2H_5$ | H | 184-187 |
| 56 | 3-Cl, $4-OCH_3$ | H | $CH_3$ | H | |
| 57 | 3-Cl, $4-OCH_3$ | H | $n-C_3H_7$ | H | |
| 58 | 4-cyclo-$C_6H_{11}$ | H | $C_2H_5$ | H | 168-172 |
| 59 | 4-Cyclo-$C_6H_{11}$ | H | Cyclo-$C_3H_5$ | H | |
| 60 | 4-Cyclo-$C_6H_{11}$ | H | $CH_3-CH=CH-$ | $CH_3$ | |
| 61 | $4-O-CF_2CHF-CF_3$ | H | $C_2H_5$ | H | 106-110 |
| 62 | $4-O-CF_2CHF-CF_3$ | H | Cyclo-$C_3H_5$ | H | |
| 63 | $4-O-CF_2CHF-CF_3$ | Cl | $C_2H_5$ | H | |
| 64 | $4-O-CF_2CHClF$ | H | $C_2H_5$ | H | |
| 65 | $4-O-CF_2CHClF$ | Cl | $C_2H_5$ | H | |
| 66 | $4-O-CF_2CHClF$ | Br | $C_2H_5$ | H | |
| 67 | $4-OCF_3$ | H | $C_2H_5$ | H | 201-203 |
| 68 | $4-OCF_3$ | Cl | $C_2H_5$ | H | |
| 69 | $4-OCF_3$ | Br | $C_2H_5$ | H | |
| 70 | $4-OCF_3$ | H | Cyclo-$C_3H_5$ | H | 227-230 |
| 71 | $4-OCF_3$ | H | $CH_2=C(CH_3)-$ | H | |
| 72 | $4-OCF_3$ | H | $C_3H_7(CH_3)_2C-$ | H | |

| Verbindung Nr. | $X_n$ | $R^3$ | $R^2$ | $R^1$ | Fp [°C] |
|---|---|---|---|---|---|
| 73 | 4-$OCF_3$ | H | $C_3H_7$-CH($CH_3$)- | H | |
| 74 | 4-$CF_3$ | H | 2,4-$Cl_2$-$C_6H_3$-O-$CH_2$- | H | 165-167 |
| 75 | 4-Cl | H | $C_2H_5$ | $CH_3$ | 140-143 |
| 76 | 4-$OCHF_2$ | Cl | $C_2H_5$ | H | |
| 77 | 4-$OCHF_2$ | Br | $C_2H_5$ | H | |
| 78 | 4-$OCHF_2$ | H | 2,4-$Cl_2$-$C_6H_3$-O-$CH_2$- | H | 140-143 |
| 79 | 4-$OCHF_2$ | H | $C_3H_7$-C($CH_3$)$_2$- | | $n_D^{25}$ 1,5330 |
| 80 | 4-$OCHF_2$ | H | $C_3H_7$-CH($CH_3$)- | H | 96- 98 |
| 81 | 4-$OCHF_2$ | H | n-$C_3H_7$ | H | |
| 82 | 2,4-$Cl_2$ | H | $C_2H_5$ | H | 171-173 |
| 83 | 2,4-$Cl_2$ | H | Cyclo-$C_3H_5$ | H | 217-220 |
| 84 | 2,5-$Cl_2$ | H | Cyclo-$C_3H_5$ | H | 197-212 |
| 85 | 2,5-$Cl_2$ | H | $C_2H_5$ | H | 199-200 |
| 86 | 4-Br | H | $C_2H_5$ | H | 199-203 |
| 87 | 4-Br | H | Cyclo-$C_3H_5$ | H | 233-235 |
| 88 | 2,4-$F_2$ | H | Cyclo-$C_3H_5$ | H | 206-211 |
| 89 | 2,4-$F_2$ | H | $C_2H_5$ | H | 165-166 |
| 90 | 2,4-($OCHF_2$)$_2$ | H | $C_2H_5$ | H | 100-105 |
| 91 | 2,4-($OCHF_2$)$_2$ | H | Cyclo-$C_3H_5$ | H | 148-150 |
| 92 | 2-F | H | Cyclo-$C_3H_5$ | H | 165-167 |
| 93 | 2-F | H | $C_2H_5$ | H | 142-144 |
| 94 | 2,3,4-$Cl_3$ | H | $C_2H_5$ | H | 151-157 |
| 95 | 2,3,4-$Cl_3$ | H | Cyclo-$C_3H_5$ | H | 187-190 |
| 96 | 2-$OCH_3$, 3,5-$Cl_2$ | H | Cyclo-$C_3H_5$ | H | |
| 97 | 2-$OCH_3$, 3,5-$Cl_2$ | H | $C_2H_5$ | H | |
| 98 | 2-$OCH_3$, 4-$CH_3$ | H | $C_2H_5$ | H | |
| 99 | 2-$OCH_3$, 4-$CH_3$ | H | Cyclo-$C_3H_5$ | H | |

| Verbindung Nr. | $X_n$ | $R^3$ | $R^2$ | $R^1$ | Fp [°C] |
|---|---|---|---|---|---|
| 100 | 4-$OCH_3$, 2-$CH_3$ | H | Cyclo-$C_3H_5$ | H | |
| 101 | 4-$OCH_3$, 2-$CH_3$ | H | $C_2H_5$ | H | |
| 102 | 4-$OCHF_2$, 2-$CH_3$ | H | $C_2H_5$ | H | 137-138 |
| 103 | 4-$OCHF_2$, 2-$CH_3$ | H | Cyclo-$C_3H_5$ | H | |
| 104 | 2-$OCHF_2$, 4-$CH_3$ | H | Cyclo-$C_3H_5$ | H | |
| 105 | 2-$OCHF_2$, 4-$CH_3$ | H | $C_2H_5$ | H | 110-125 |
| 106 | 4-$OCHF_2$, 2-Cl | H | $C_2H_5$ | H | |
| 107 | 4-$OCHF_2$, 2-Cl | H | Cyclo-$C_3H_5$ | H | |
| 108 | 4-Cl, 2-$OCHF_2$ | H | Cyclo-$C_3H_5$ | H | 152-163 |
| 109 | 4-Cl, 2-$OCHF_2$ | H | $C_2H_5$ | H | 150-161 |
| 110 | 4-Cl, 2-$OCH_3$ | H | $C_2H_5$ | H | |
| 111 | 4-Cl, 2-$OCH_3$ | H | Cyclo-$C_3H_5$ | H | |
| 112 | 2-Cl, 4-$OCH_3$ | H | Cyclo-$C_3H_5$ | H | |
| 113 | 2-Cl, 4-$OCH_3$ | H | $C_2H_5$ | H | |
| 114 | 3,5-$Cl_2$ | H | $C_2H_5$ | H | |
| 115 | 3,5-$Cl_2$ | H | Cyclo-$C_3H_5$ | H | |
| 116 | 2,6-$Cl_2$ | H | Cyclo-$C_3H_5$ | H | |
| 117 | 2,6-$Cl_2$ | H | $C_2H_5$ | H | |
| 118 | 2,4-$Cl_2$ | Br | $C_2H_5$ | H | 151-153 |
| 119 | 4-t-$C_4H_9$ | H | $C_2H_5$ | H | 198-200 |
| 120 | 4-t-$C_4H_9$ | H | Cyclo-$C_3H_5$ | H | 199-202 |
| 121 | 3-$CF_3$ | H | $C_2H_5$ | H | 157-160 |

phenon, Alkylphenolpolyglykolether, Tributylphenylpolyglykolether, Alkalarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gew.%, vorzugsweise zwischen 0,5 und 90 Gew.%, Wirkstoff.

Beispiele für Formulierungen sind :

I. Man vermischt 90 Gewichtsteile der Verbindung Nr. 18 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteile Calciumsalz der Dodecylbenzolsulfonsäure und 5 Gewichtsteile des Anlagerungsproduktes von 40 Mol Ethylenoxid and 1 Mol Ricinusöl besteht. Durch Ausgießen und feines Verteilen der Lösung in 100 000 Gewichtsteilen Wasser erhält man eine Wäßrige Dispersion, die 0,02 Gew.% des Wirkstoffs enthält.

III. 20 Gewichtsteile der Verbindung Nr. 5 werden in einer Mischung gelöst, die aus 40 Gewichtsteilen Cyclohexanon, 30 Gewichtsteilen Isobutanol, 20 Gewichtsteile des Anlagerungsproduktes vo 7 Mol Ethylenoxid an 1 Mol Isooctylphenol und 10 Gewichtsteile des Anlagerungsproduktes von 40 Mol Ethylen oxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IV. 20 Gewichtsteile des Wirkstoffs Nr. 26 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöls besteht. Durch Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 10 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 12 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 4 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile des Wirkstoffs Nr. 3 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teile eines paraffinischen Mineralöls innig vermischt. Man erhält eine stabile ölige Dispersion.

Die Applikation der Wirkstoffe bzw. der Mittel kann im Vorauflaufverfahren oder im Nachauflaufverfahren erfolgen. Sind die Wirkstoffe für gewisse Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (Post-directed, lay-by).

Die Aufwandmengen an Wirkstoff betragen je nach Jahreszeit, Zielpflanzen und Wachstumsstadium 0,1 bis 5 kg/ha, vorzugsweise 0,25 bis 3 kg/ha.

Die Wirkung der Thiazolylamide der Formel Ia auf das Pflanzenwachstum läßt sich durch Gewächshausversuche zeigen :

Als Kulturgefäße dienen Plastikblumentöpfe mit 300 $cm^3$ Inhalt und lehmigem Sand mit etwa 3,0 % Humus als Substrat. Die Samen der Testpflanzen werden nach Arten getrennt flach eingesät. Bei Vorauflaufbehandlung werden die Wirkstoffe unmittelbar danach auf die Erdoberfläche aufgebracht. Sie werden hierbei in Wasser als Verteilungsmittel suspendiert oder emulgiert und mittels fein verteilender Düsen gespritzt. Die Aufwandmengen betragen 3,0 kg/ha.

Nach dem Aufbringen der Mittel werden die Gefäße leicht beregnet, um Keimung und Wachstum in Gang zu bringen, Danach deckt man die Gefäße mit durchsichtigen Plastikhauben ab, bis die Pflanzen angewachsen sind. Diese Abdeckung bewirkt ein gleichmäßiges Keimen der Testpflanzen, sofern dies nicht durch die Wirkstoffe beeinträchtigt wird.

Zum Zweck der Nachauflaufbehandlung zieht man die Testpflanzen je nach Wuchsform erst bis zu einer Wuchshöhe von 3 bis 15 cm an und behandelt sie danach. Die Sojapflanzen werden in einem mit Torfmull (peat) angereicherten Substrat angezogen. Zur Nachauflaufbehandlung werden entweder direkt gesäte und in den gleichen Gefäßen aufgewachsene Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung betragen 0,5 bis 3 kg Wirkstoff/ha. Eine Abdeckung unterbleibt bei der Nachauflaufbehandlung.

Die Versuchsgefäße werden im Gawächshaus aufgestellt, wobei für wärmeliebende Arten wärmere Bereiche (20 bis 35 °C) und für solche gemäßigter Klimate 10 bis 25 °C bevorzugt werden. Die Versuchsperiode erstreckt sich über 2 bis 4 Wochen. Während dieser Zeit werden die Pflanzen verpflegt und ihre Reaktion auf die einzelnen Behandlungen wird ausgewertet. Bewertet wird nach einer Skala von 0 bis 100. Dabei bedeutet 100 kein Aufgang der Pflanzen bzw. völlige Zerstörung zumindest der oberirdischen Teile.

In den Versuchen werden folgende Pflanzenarten verwendet:

Amaranthus spp. (Fuchsschwanzarten), Arachis hypogaea (Erdnuß), Avena sativa (Hafer), Chenopodium album (Weißer Gänsefuß), Euphorbia geniculata (Südamerik, Wolfsmilch), Glycine max. (Sojabohnen), Ipomoea spp. (Prunkwindearten), Lamium amplexicaule (Stengelumfassende Taubnessel), Lolium multiflorum (Ital. Raygras), Mercurialis annua (Einjähriges Bingelkraut), Sinapis alba (Weißer Senf), Solanum nigrum (Schwarzer Nachtschatten), Triticum aestivum (Weizen).

Bei Vorauflaufanwendung zeigt beispielsweise die Verbindung Nr. 42 eine nennenswerte herbizide Wirkung gegen einen Vertreter der Grasarten wie auch gegen eine dikotyle Beispielspflanze.

Bei Nachauflaufanwendung bekämpfen die beispielhaft ausgewählten Verbindungen Nr. 9, 13, 43 und 45 bei einer Aufwandmenge von 3,0 kg Wirkstoff/ha breitblättrige Pflanzen. Die selektive Bekämpfung breitblättriger unerwünschter Pflanzen in Kulturen wie Erdnüsse, Soja und Weizen zeigen mit 0,5 kg/ha Wirkstoff Nachauflaufanwendung die Verbindungen Nr. 18 und 45.

Die Wirkstoffe Nr. 47 und 80 eignen sich mit 1,0 kg/ha a. S. im Nachauflaufverfahren zur Bekämpfung eines breiten Spektrums unerwünschter Pflanzen in der Beispielkultur Weizen, ohne diese zu schädigen. Die Mittel sind selektiv.

Erdnüsse als breitblättrige Beispielkultur werden bei Nachauflaufanwendung von 1,0 kg/ha a. S. von Wirkstoff 61 nur unwesentlich beeinflußt. Gegen eine Reihe breitblättriger unerwünschter Pflanzen läßt sich hingegen ein guter Bekämpfungserfolg erzielen.

Unerwünschte breitblättrige Pflanzen, beispielhaft Amaranthus retroflexus und Mercurialis annua, lassen sich in Sonnenblumen bei Anwendung von 1,0 kg/ha des Wirkstoffs 45 gut bekämpfen. DE-OS 16 42 352, Beispiel 10 schädigt hingegen die Kulturpflanze im Nachauflaufverfahren erheblich und zeigt deutlich geringere herbizide Wirkung.

Wirkstoff 45 eignet sich zur Bekämpfung von Sesbania exaltata im Nachauflaufverfahren, ohne die Kulturpflanze Reis nennenswert zu schädigen. Hingegen läßt sich die Verbindung von Beispiel 10 der DE-OS 16 42 352 nicht zu diesem Zweck verwenden.

In Anbetracht der guten Verträglichkeit der Wirkstoffe und der Vielseitigkeit der Applikationsmethoden können die Thiazolylamide der Formel Ia oder diese enthaltende Mittel außer bei den in den Gawächshausversuchen getesteten Nutzpflanzen noch in einer weiteren großen Zahl von Kulturpflanzen zur Beseitigung unerwünschten Pflanzenwuchses eingesetzt werden.

In Betracht kommen beispielsweise die folgenden Kulturen:

| Botanischer Name | Deutscher Name |
|---|---|
| Allium cepa | Küchenzwiebel |
| Ananas comusus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |

| | |
|---|---|
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| cucumis melo | Melone |
| Cucumis sativus | Gurke |
| Cynodon dactylon | Bermudagras |
| Daucus carota | Möhre |
| Elaeis guineensis | Ölpalme |
| Euphorbia heterophylla | Wolfsmilch-Art |
| Fragaria vesca | Erdbeere |
| Glycine max | Sojabohne |
| Gossypium hirsutum (Gossypium arboreum) | Baumwolle |
| Gossypium herbaceum Gossypium vitifolium) | |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon | Tomate |
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum Officinarum | Zuckerrohr |
| Sesbania exaltata | Turibaum |
| Secale cereale | Roggen |
| Sasamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preißelbeere |

| | |
|---|---|
| Veronica spp. | Ehrenpreisarten |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die Thiazolylamide der Formel Ia Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4 H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem ist es nützlich, die Thiazolylamide der Formel Ia allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösugen, welche zur Behebung von Ernährungs- und Spurenelementmängeln eingesetzt werden. Es können auch nichtphytotoxische Öle und Ölkonzentrate zugesetzt werden.

## Patentansprüche

1. Thiazolylamide der Formel

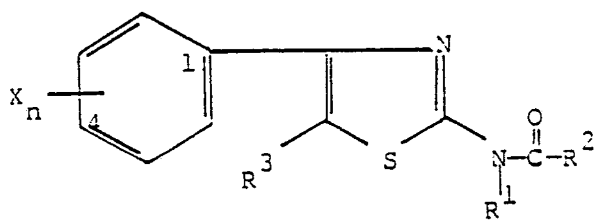

(I)

in der

$R^1$ Wasserstoff oder $C_1$-$C_4$-Alkyl,

$R^2$ $C_2$-$C_4$-Alkyl, $C_2$-$C_4$-Alkenyl, $C_2$-$C_4$-Alkinyl oder $C_3$-$C_7$-Cycloalkyl,

$R^3$ Wasserstoff, $C_1$-$C_4$-Alkyl oder Halogen,

X Halogen, $C_1$-$C_6$-Alkoxy, $C_1$-$C_6$-Halogenalkoxy, $C_1$-$C_6$-Alkyl, $C_1$-$C_6$-Halogenalkyl, $C_3$-$C_6$-Cycloalkyl, $C_1$-$C_6$-Alkylthio, Nitro, Cyano, Benzyloxy gegebenenfalls durch Halogen substituiertes Phenyl oder gegebenenfalls durch Halogen und/oder Halogenmethyl substituiertes Phenoxy und

n 1, 2, 3 oder 4 bedeuten,

2. Thiazolylamide der Formel I gemäß Anspruch 1, dadurch gekennzeichnet, daß $R^1$ Wasserstoff und $R^2$ $C_2$-$C_4$-Alkyl, $C_2$-$C_4$-Alkenyl oder $C_3$-$C_5$-Cycloalkyl bedeuten.

3. Thiazolylamide der Formel I gemäß Anspruch 1, dadurch gekennzeichnet, daß X $C_1$-$C_4$-Halogenalkoxy bedeutet.

4. Verfahren zur Herstellung von Thiazolylamiden der Formel I gemäß Anspruch 1, dadurch gekennzeichnet, daß man ein substituiertes Amin der Formel II

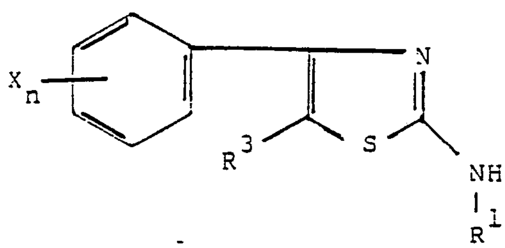

(II)

mit einem Säureanhydrid der Formel III

$R^2$—CO—O—CO—R2 (III)

in der $R^2$ die obengenannten Bedeutungen hat,
in an sich üblicher Weise umsetzt.

5. Verfahren zur Herstellung von Thiazolylamiden der Formel I gemäß Anspruch 1, dadurch gekennzeichnet, daß man ein substituiertes Amin der Formel

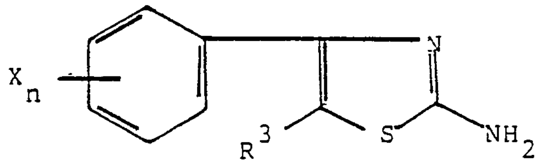

(IIa)

in der $R^3$ und $X_n$ die im Anspruch 1 genannten Bedeutungen haben

a) mit Phosgen, Oxalylchlorid oder Diphosgen und anschließend mit einer Säure der Formel

$R^2$—COOH (VI)

in der $R^2$ die im Anspruch 1 genannte Bedeutung hat, oder

b) mit einem Säurehalogenid der Formel

Hal—CO—$R^2$ (IV)

in der $R^2$ die im Anspruch 1 genannten Bedeutungen hat und Hal für Halogen, vorzugsweise Clor, steht, umsetzt.

6. Herbizides Mittel, enthaltend ein Thiazolylamid der Formel

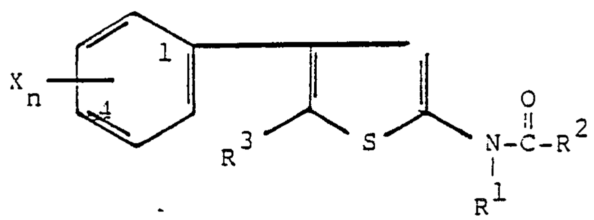

(Ia)

wobei

$R^1$ Wasserstoff oder $C_1$-$C_4$-Alkyl,

$R^2$-Wasserstoff

-$C_3$-$C_7$-Cycloalkyl

-$C_1$-$C_6$-Alkyl, $C_2$-$C_6$-Alkenyl oder $C_2$-$C_6$-Alkinyl, wobei diese Gruppen durch Halogen, $C_1$-$C_4$-Alkoxy, $C_1$-$C_4$-Alkylthio oder Phenoxy substituiert sein können und wobei die Phenoxygruppe ihrerseits durch Halogen oder $C_1$-$C_4$-Alkyl substituiert sein kann,

$R^3$ Wasserstoff, $C_1$-$C_4$-Alkyl oder Halogen,

X Wasserstoff, Halogen, $C_1$-$C_6$-Alkoxy, $C_1$-$C_6$-Halogenalkoxy, $C_1$-$C_6$-Alkyl, $C_1$-$C_6$-Halogenalkyl, $C_3$-$C_6$-Cycloalkyl, $C_1$-$C_6$-Alkylthio, Nitro, Cyano, Benzyloxy, gegebenenfalls durch Halogen substituiertes Phenyl oder gegebenenfalls durch Halogen und/oder Halogenmethyl substituiertes Phenoxy und

n 1, 2, 3 oder 4 bedeuten ;

7. Herbizid, enthaltend inerte Zusatzstoffe und ein Thiazolylamid der Formel Ia gemäß Anspruch 6.

8. Verfahren zur Herstellung eines herbiziden Mittels gemäß Anspruch 7, dadurch gekennzeichnet, daß man inerte Zusatzstoffe mit 0,1 bis 95 Gew.% eines Thiazolylamids der Formel Ia gemäß Anspruch 6 vermischt.

9. Verfahren zur Bekämpfung von unerwünschtem Pflanzenwuchs, dadurch gekennzeichnet, daß man die unerwünschten Pflanzen oder von unerwünschtem Pflanzenwuchs freizuhaltende Flächen mit einer herbizid wirksamen Menge eines Thiazolylamids der Formel Ia gemäß Anspruch 6 behandelt.

## Claims

1. A thiazolylamide of the formula

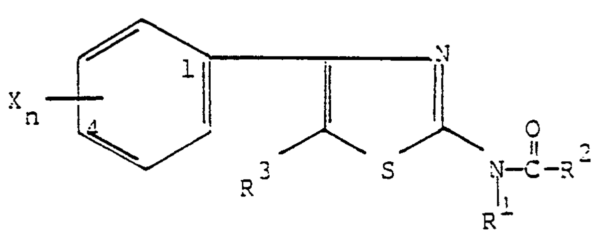

(I)

where $R^1$ is hydrogen or $C_1$-$C_4$-alkyl, $R^2$ is $C_2$-$C_4$-alkyl, $C_2$-$C_4$-alkenyl, $C_2$-$C_4$-alkynyl or $C_3$-$C_7$-cycloalkyl, $R^3$ is hydrogen, $C_1$-$C_4$-alkyl or halogen, X is halogen, $C_1$-$C_6$-alkoxy, $C_1$-$C_6$-haloalkoxy, $C_1$-$C_6$-alkyl, $C_1$-$C_6$-haloalkyl, $C_3$-$C_6$-cycloalkyl, $C_1$-$C_6$-alkylthio, nitro, cyano, benzyloxy, unsubstituted or halogen-substituted phenyl, or phenoxy which is unsubstituted or substituted by halogen and/or halomethyl, and n is 1, 2, 3 or 4.

2. A thiazolylamide of the formula I as claimed in claim 1, where $R^1$ is hydrogen and $R^2$ is $C_2$-$C_4$-alkyl, $C_2$-$C_4$-alkenyl or $C_3$-$C_5$-cycloalkyl.

3. A thiazolylamide of the formula I as claimed in claim 1, where X is $C_1$-$C_4$-haloalkoxy.

4. A process for preparing a thiazolylamide of the formula I as claimed in claim 1, wherein a substituted amine of the formula II

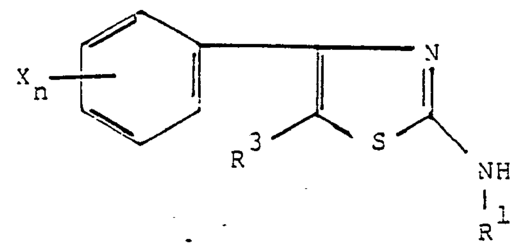

(II)

is reacted in a conventional manner with an acid anhydride of the formula III

$$R^2—CO—O—CO—R^2 \quad (III)$$

where $R^2$ has the above meanings.

5. A process for preparing a thiazolylamide of the formula I as claimed in claim 1, wherein a substituted amine of the formula

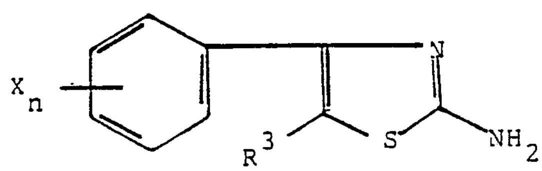

(IIa)

where $R^3$ and $X_n$ have the meanings given in claim 1 is reacted

a) with phosgene, oxalyl chloride or diphosgene, and subsequently with an acid of the formula

$$R^2—COOH \quad (VI)$$

where $R^2$ has the meaning given in claim 1, or

b) with an acid halide of the formula

$$Hal—CO—R^2 \quad (IV)$$

where $R^2$ has the meanings given in claim 1 and Hal is halogen, preferably chlorine.

6. A herbicide containing a thiazolylamide of the formula

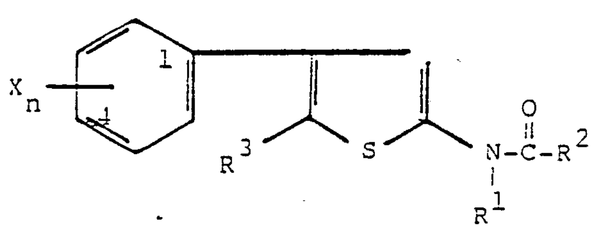

(Ia)

where $R^1$ is hydrogen or $C_1$-$C_4$-alkyl, $R^2$ is hydrogen or $C_3$-$C_7$-cycloalkyl or is $C_1$-$C_6$-alkyl, $C_2$-$C_6$-alkenyl or $C_2$-$C_6$-alkynyl, each of which is unsubstituted or substituted by halogen, $C_1$-$C_4$-alkoxy, $C_1$-$C_4$-alkylthio

or phenoxy, phenoxy being in turn unsubstituted or substituted by halogen or $C_1$-$C_4$-alkyl, $R^3$ is hydrogen, $C_1$-$C_4$-alkyl or halogen, X is hydrogen, halogen, $C_1$-$C_6$-alkoxy, $C_1$-$C_6$-haloalkoxy, $C_1$-$C_6$-alkyl, $C_1$-$C_6$-haloalkyl, $C_3$-$C_6$-cycloalkyl, $C_1$-$C_6$-alkylthio, nitro, cyano, benzyloxy, unsubstituted or halogen-substituted phenyl, or phenoxy which is unsubstituted or substituted by halogen and/or halomethyl, and n is 1, 2, 3 or 4.

7. A herbicide containing inert additives and a thiazolylamide of the formula Ia as claimed in claim 6.

8. A process for preparing a herbicide as claimed in claim 7, wherein inert additives are mixed with from 0.1 to 95 % by weight of a thiazolylamide of the formula Ia as claimed in claim 6.

9. A method for controlling undesirable plant growth, wherein the undesirable plants or the areas to be kept free from undesirable plant growth are treated with a herbicidally effective amount of a thiazolylamide of the formula Ia as claimed in claim 6.

**Revendications**

1. Thiazolylamide de formule

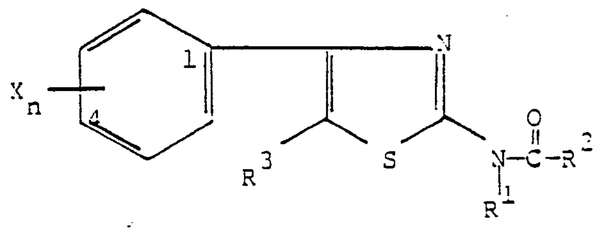

(I)

dans laquelle

$R^1$ représente hydrogène ou alkyle en $C_1$-$C_4$

$R^2$, alkyle en $C_2$-$C_4$, alcényle en $C_2$-$C_4$, alcinyle en $C_2$-$C_4$, ou cycloalkyle en $C_3$-$C_7$,

$R^3$, hydrogène, alkyle en $C_1$-$C_4$ ou halogène

X, halogène, alcoxy en $C_1$-$C_6$, halogénoalcoxy en $C_1$-$C_6$, alkyle en $C_1$-$C_6$, halogénoalkyle en $C_1$-$C_6$, cycloalkyle en $C_3$-$C_6$, alkylthio en $C_1$-$C_6$, nitro, cyano, benzyloxy, phényle éventuellement substitué par halogène, ou phénoxy éventuellement substitué par halogène et/ou halogénométhyle, et

n, représente 1, 2, 3 ou 4.

2. Thiazolylamide de formule I selon la revendication 1, caractérisée par le fait que $R^1$ représente hydrogène et $R^2$, alkyle en $C_2$-$C_4$, alcényle en $C_2$-$C_4$ ou cycloalkyle en $C_3$-$C_5$.

3. Thiazolylamide de formule I selon la revendication 1, caractérisé par le fait que X représente halogénoalcoxy en $C_1$-$C_4$.

4. Procédé de préparation de thiazolylamides de formule I selon la revendication 1, caractérisé par le fait que l'on fait réagir, de manière connue en soi, une amine substituée de formule II

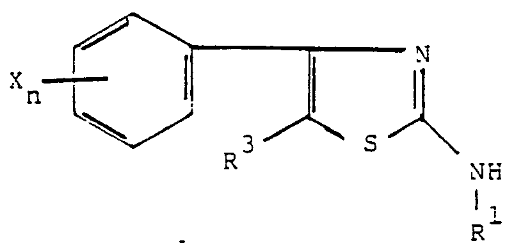

(II)

avec un anhydride d'acide de formule III

$$R^2\text{—CO—O—CO—}R^2 \qquad \text{(III)}$$

dans laquelle $R^2$ a la signification sus-indiquée.

5. Procédé de préparation de thiazolylamides, caractérisé par le fait que l'on fait réagir une amine substituée de formule

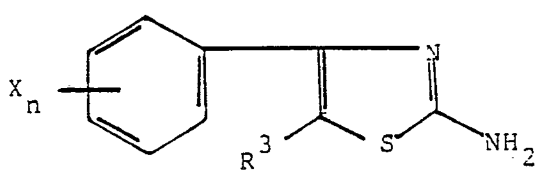

(IIa)

dans laquelle $R^3$ et $X_n$ ont les significations indiquées dans la revendication 1.

a) avec du phosgène, chlorure d'oxalyle ou diphosgène et ensuite avec un acide de formule

$$R^2—COOH \quad (VI)$$

dans laquelle $R^2$ a la signification indiquée dans la revendication 1, ou

b) avec un halogénure d'acide de formule

$$Hal—CO—R^2 \quad (IV)$$

dans laquelle $R^2$ a la signification indiquée dans la revendication 1 et Hal est mis pour halogène, de préférence chlore.

6. Agent herbicide, contenant un thiazolylamide de formule

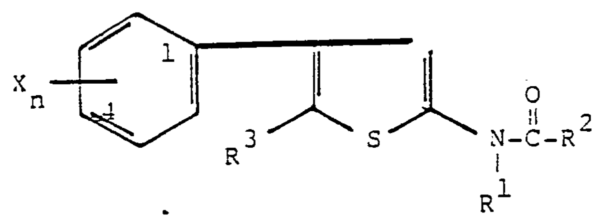

(Ia)

où

$R^1$ représente hydrogène ou alkyle en $C_1$-$C_4$

$R^2$, hydrogène, cycloalkyle en $C_3$-$C_7$, alkyle en $C_1$-$C_6$, alcényle en $C_2$-$C_6$ ou alcinyle en $C_2$-$C_6$, ces groupes pouvant être substitués par halogène, alcoxy en $C_1$-$C_4$, alkylthio en $C_1$-$C_4$ ou phénoxy et le groupe phénoxy, à son tour, pouvant être substitué par halogène ou alkyle en $C_1$-$C_4$

$R^3$, hydrogène, alkyle en $C_1$-$C_4$ ou halogène

X, hydrogène, halogène, alcoxy en $C_1$-$C_6$, halogénoalcoxy en $C_1$-$C_6$, alkyle en $C_1$-$C_6$, halogénoalkyle en $C_1$-$C_6$, cycloalkyle en $C_3$-$C_6$, alkylthio en $C_1$-$C_6$, nitro, cyano, benzyloxy, phényle éventuellement substitué par halogène, ou phénoxy éventuellement substitué par halogène et/ou halogénométhyle, et

n, représente 1, 2, 3 ou 4.

7. Herbicide contenant des additifs inertes et un triazolylamide de formule la selon la revendication 6.

8. Procédé de préparation d'un agent herbicide selon la revendication 7, caractérisé par le fait que l'on mélange des additifs inertes avec 0,1 à 95 % en poids un triazolylamide de formule la selon la revendication 6.

9. Procédé de lutte contre la croissance indésirable des plantes, caractérisé par le fait que l'on traite les plantes indésirables ou les surfaces à maintenir exemptes d'une croissance indésirable des plantes, avec une quantité efficace du point de vue herbicide d'un triazolylamide de formule la selon la revendication 6.